(19) Europäisches Patentamt
European Patent Office
Office européen des brevets

(11) **EP 0 864 314 A2**

(12) **EUROPÄISCHE PATENTANMELDUNG**

(43) Veröffentlichungstag:
16.09.1998 Patentblatt 1998/38

(51) Int. Cl.$^6$: **A61K 7/06**

(21) Anmeldenummer: 97121198.2

(22) Anmeldetag: 03.12.1997

(84) Benannte Vertragsstaaten:
AT BE CH DE DK ES FI FR GB GR IE IT LI LU MC NL PT SE
Benannte Erstreckungsstaaten:
AL LT LV MK RO SI

(30) Priorität: 15.03.1997 DE 19710874

(71) Anmelder:
Wella Aktiengesellschaft
64274 Darmstadt (DE)

(72) Erfinder:
• Titze, Hans-Jürgen
  64401 Grosse-Bieberau (DE)
• Karlen, Thomas Dr.
  3013 Bern (DE)
• Birkel, Susanne Dr.
  64380 Rossdorf (DE)
• Schmenger, Jürgen
  64331 Weiterstadt (DE)

(54) **Verwendung natürlicher Öle in Haarbehandlungsmitteln**

(57) Gegenstand der Erfindung ist die Verwendung von mindestens einem der natürlichen Öle Krambeöl und Leindotteröl zur Haarbehandlung sowie Haarbehandlungsmittel, welche in einer geeigneten kosmetischen Grundlage mindestens eines dieser Öle enthalten.

Durch Zusatz von Krambeöl und/oder Leindotteröl werden die Elastizität, die Sprungkraft und der Haarglanz erheblich verbessert. Polymerfilme mit einem Gehalt an Leindotteröl oder Krambeöl weisen eine erhöhte Resistenz gegenüber Feuchtigkeit auf.

## Beschreibung

Gegenstand der Erfindung ist die Verwendung von mindestens einem der natürlichen Öle Krambeöl und Leindotteröl zur Haarbehandlung sowie Haarbehandlungsmittel, welche in einer geeigneten kosmetischen Grundlage mindestens eines dieser Öle enthalten.

Ein ansprechendes äußeres Erscheinungsbild wurde schon immer als sehr wichtig angesehen. Eine besondere Rolle spielt dabei die Frisur. Basis für ein ansprechendes Äußeres ist gut frisiertes und gepflegtes Haar. Für die Reinigung und Pflege des Haares gibt es eine ganze Reihe von Haarbehandlungsmitteln wie Shampoos, Kuren, Spülungen, Spitzenfluids sowie Mittel zur Verformung und Stabilisierung der Frisur, die allgemein als Stylingmittel bekannt sind.

Es sind bereits zahlreiche Produkte bekannt, welche den Haaren durch Polymerzusatz Halt, Volumen, Elastizität, Sprungkraft und Glanz verleihen. Diese Stylingprodukte erleichtern als Gel die Formgebung, verbessern als Haarspray den Stand und als Festigerschaum das Volumen des Haares. Darüber hinaus sollen Stylingprodukte dem Haar neben natürlicher Sprungkraft und Elastizität einen natürlichen Glanz verleihen. Der Glanz des Haares ist ein Spiegel für Schönheit, Gesundheit und Vitalität.

Die Schönheit des Haares wird vor allem durch zwei Parameter beeinflußt, nämlich durch die Farbtiefe und den Glanz. Die Farbtiefe wird bestimmt durch den Weißanteil des Lichtes. Ist der Weißanteil hoch, ist die Farbtiefe gering.

Der Glanz wird bestimmt durch den Anteil des gerichtet reflektierten Lichtes im Verhältnis zum diffus reflektierten Licht. An einer glatten Oberfläche wird gerichtetes Licht reflektiert und die Oberfläche (das Haar) erscheint dann glänzend. Welche Zusatzstoffe zu Haarbehandlungsmitteln den Glanz des damit behandelten Haares besonders stark erhöhen, kann nicht vorausgesagt werden.

Es stellt sich deshalb die Aufgabe, bei herkömmlichen Haarbehandlungsmitteln durch geeignete Zusätze die Elastizität, die Sprungkraft und vor allem den Glanz des menschlichen Haares weiter zu erhöhen. Diese Aufgabe läßt sich erfindungsgemäß besonders eindrucksvoll durch die Verwendung von Krambeöl oder Leindotteröl lösen.

Es wurde nun gefunden, daß ein Mittel zur Behandlung von Haaren die vorteilhaften Eigenschaften der bisher bekannten Produkte noch übertrifft, wenn es unter Verwendung mindestens eines natürlichen Öls, welches ausgewählt ist aus Krambeöl und Leindotteröl, hergestellt ist.

Durch Zusatz von Krambeöl und/oder Leindotteröl werden die Elastizität, die Sprungkraft und der Haarglanz erheblich verbessert. Führt man eine Haarbehandlung mit Stylingprodukten durch, die mit oder ohne Zugabe von Krambeöl und/oder Leindotteröl hergestellt waren, zeigen die unter Verwendung von Krambeöl und/oder Leindotteröl enthaltenden Präparate behandelten Haare bei einer sensorischen Beurteilung ein deutlich elastischeres Anfühlen. Anwendungstechnische Versuche beim Halbseitenvergleich zeigen, daß die Zugabe von Krambeöl und/oder Leindotteröl zum Stylingprodukt den Glanz der damit behandelten Haare deutlich erhöht.

Es war überraschend, daß gerade durch den Zusatz von Krambeöl und/oder Leindotteröl zu herkömmlichen Stylingprodukten eine bemerkenswerte Erhöhung des Haarglanzes erreicht werden kann.

Krambeöl und Leindotteröl werden durch Kaltpressung mit einer Ölpresse aus der Ölpflanze Krambe (Grambe abyssinica) beziehungsweise aus den Samen von Leindotter (Camelina, Öldotter) gewonnen und können ohne weitere Reinigung oder chemische Nachbehandlung eingesetzt werden. Die Öle zeichnen sich durch einen hohen Anteil an C16- bis C24-Fettsäuren, insbesondere durch einen hohen Gehalt an ungesättigten Fettsäuren wie Oel-, Linol-, Linolen-, Eicosen-, Eicosadien-, Eruca-, Docosadien- und Nervonsäure aus. So hat Krambeöl einen Erucasäureanteil von 55 bis 65 Prozent und Leindotteröl einen Eicosen- bzw. Eicosadiensäureanteil von 15 bis 25 Prozent.

In einem erfindungsgemäßen Haarbehandlungsmittel ist das Krambeöl und/oder Leindotteröl in einer Menge von vorzugsweise 0,01 bis 10 Gewichtsprozent, besonders bevorzugt in einer Menge von 0,05 bis 3,0 Gewichtsprozent in einer geeigneten kosmetischen Grundlage enthalten.

In einer besonderen Ausführungsform wird das Krambeöl und/oder Leindotteröl zusammen mit mindestens einem synthetischen oder natürlichen, nichtionischen, kationischen, anionischen oder amphoteren, filmbildenden und haarfestigenden Polymeren eingesetzt. Ein derartiges Polymeres, das in Mengen von 0,01 bis 50 Gewichtsprozent, vorzugsweise in Mengen von 0,5 bis 20 Gewichtsprozent im Haarbehandlungsmittel enthalten ist, kann auch aus einem Gemisch mehrerer Polymerer bestehen und durch den Zusatz weiterer Polymerer mit verdickender Wirkung in seinen haarfestigenden Eigenschaften noch modifiziert werden.

Messungen von Wasserdampfaufnahmen zeigen, daß Polymerfilme mit einem Gehalt an Leindotteröl oder Krambeöl eine deutlich erhöhte Resistenz gegenüber Feuchtigkeit aufweisen.

Unter filmbildenden, haarfestigenden Polymeren werden erfindungsgemäß solche Polymere verstanden, die bei Anwendung in 0,01 bis 5%-iger wäßriger, alkoholischer oder wäßrig-alkoholischer Lösung in der Lage sind, auf dem Haar einen Polymerfilm abzuscheiden und auf diese Weise das Haar zu festigen.

Als geeignete synthetische, nichtionische, filmbildende, haarfestigende Polymere können in dem erfindungsgemäßen Haarbehandlungsmittel Homopolymere des Vinylpyrrolidons, Homopolymere des N-Vinylformamids, Copolymerisate aus Vinylpyrrolidon und Vinylacetat, Terpolymere aus Vinylpyrrolidon, Vinylacetat und Vinylpropionat, Polyacrylamide, Polyvinylalkohole, oder Polyethylenglykole mit einem Molekulargewicht von 800 bis 20.000 g/mol eingesetzt werden.

Unter den geeigneten synthetischen, filmbildenden anionischen Polymeren sind zu nennen Crotonsäure-Vinylacetat-Copolymere und Terpolymere aus Acrylsäure, Ethylacrylat und N-t-Butylacrylamid.

Natürliche filmbildende Polymere oder daraus durch chemische Umwandlung hergestellte Polymere können in dem erfindungsgemäßen Haarbehandlungsmittel ebenfalls eingesetzt werden. Bewährt haben sich niedermolekulares Chitosan mit einem Molekulargewicht von 30.000 bis 70.000 g/mol oder hochmolekulares Chitosan, Gemische aus Oligo-, Mono- und Disacchariden, chinesisches Balsamharz, Cellulosederivate wie Hydroxypropylcellulose mit einem Molekulargewicht von 30.000 bis 50.000 g/mol, oder Schellack in neutralisierter oder unneutralisierter Form.

Auch amphotere Polymere können in dem erfindungsgemäßen Haarbehandlungsmittel eingesetzt werden. Geeignet sind zum Beispiel Copolymere aus Octylacrylamid, t-Butylaminoethylmethacrylat sowie zwei oder mehr Monomeren aus der Gruppe Acrylsäure, Methacrylsäure und deren einfachen Estern.

Unter den kationischen Polymeren, die erfindungsgemäß eingesetzt werden können, sind Copolymere des Vinylpyrrolidons mit quaternierten Derivaten des Dialkylaminoacrylats- und -methacrylats, wie beispielsweise mit Diethylsulfat quaternierte Vinylpyrrolidon/Dimethylaminomethacrylat Copolymere zu nennen. Weitere kationische Polymere sind beispielsweise das Copolymerisat des Vinylpyrrolidons mit Vinylimidazoliummethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcarprolactam, das quaternierte Ammoniumsalz, hergestellt aus Hydroxyethylcellulose und einem mit Trimethylammonium substituierten Epoxid, das Vinylpyrrolidon/Methacrylamidopropyltrimethylammoniumchlorid Copolymer und diquaternäre Polydimethylsiloxane.

Die Konsistenz des erfindungsgemäßen Haarbehandlungsmittels kann durch den Zusatz von Verdikkern erhöht werden. Hierfür sind beispielsweise Homopolymere der Acrylsäure mit einem Molekulargewicht von 2.000.000 bis 6.000.000 g/mol geeignet. Auch Copolymere aus Acrylsäure und Acrylamid (Natriumsalz) mit einem Molekulargewicht von 2.000.000 bis 6.000.000 g/mol und Sclerotium Gum sind geeignet. Auch geeignet sind Copolymere der Acrylsäure und der Methacrylsäure.

Das erfindungsgemäße Haarbehandlungsmittel liegt im allgemeinen als wäßrige, alkoholische oder wäßrigalkoholische Lösung vor. Geeignete Lösungsmittel sind beispielsweise aliphatische Alkohole mit 1 bis 4 Kohlenstoffatomen oder ein Gemisch von Wasser mit einem der genannten Alkohole. Es können jedoch auch andere organische Lösungsmittel eingesetzt werden, wobei insbesondere unverzweigte oder verzweigte Kohlenwasserstoffe wie Pentan, Hexan, Isopentan und zyklische Kohlenwasserstoffe wie Cyclopentan und Cyclohexan zu nennen sind. Die Lösungsmittel liegen in einer Menge von 0,5 bis 90 Gewichtsprozent, bevorzugt in einer Menge von 5 bis 50 Gewichtsprozent vor.

Üblicherweise können dem erfindungsgemäßen Haarbehandlungsmittel weitere bekannte kosmetische Zusatzstoffe beigefügt werden, zum Beispiel nichtfestigende, nichtionische Polymere wie Polyethylenglykol mit einem Molekulargewicht von etwa 600 g/mol, nichtfestigende, anionische und natürliche Polymere sowie deren Mischungen in einer Menge von vorzugsweise 0,01 bis 50 Gewichtsprozent. Auch Parfümöle in einer Menge von 0,01 bis 5 Gewichtsprozent, Trübungsmittel wie Ethylenglykoldistearat in einer Menge von 0,01 bis 5 Gewichtsprozent, Netzmittel oder Emulgatoren aus den Klassen der anionischen, kationischen, amphoteren oder nichtionogenen Tenside wie Fettalkoholsulfate, ethoxylierte Fettalkohole, Fettsäurealkanolamide wie die Ester der hydrierten Rizinusölfettsäuren in einer Menge von 0,1 bis 30 Gewichtsprozent, außerdem Feuchthaltemittel, Farbstoffe, Lichtschutzmittel, Antioxidantien und Konservierungsstoffe in einer Menge von 0,01 bis 10 Gewichtsprozent.

Das erfindungsgemäße Haarbehandlungsmittel kann weiterhin durch Zusatz von Silikonpolymeren verbessert werden, wie beispielsweise Polydimethylsiloxan (INCI: Dimethicon), $\alpha$-Hydro-$\omega$-hydroxypolyoxydimethylsilylen (INCI: Dimethiconol), cyclisches Dimethylpolysiloxan (INCI: Cyclomethicon), Trimethyl(octadecyloxy)silan (INCI: Stearoxytrimethylsilan), Dimethylsiloxan/Glykol Copolymer (INCI: Dimethicon Copolyol), Dimethylsiloxan/Aminoalkylsiloxan Copolymer mit Hydroxyendgruppen (INCI: Amodimethicon), Monomethylpolysiloxan mit Laurylseitenketten und Polyoxyethylen- und/oder Polyoxypropylenendketten, (INCI: Laurylmethicon Copolyol), Dimethylsiloxan/Glykol Copolymeracetat (INCI: Dimethiconcopolyol Acetat), Dimethylsiloxan/Aminoalkylsiloxan Copolymer mit Trimethylsilylendgruppen (INCI: Trimethylsilylamodimethicon). Bevorzugte Silikonpolymere sind Dimethicone, Cyclomethicone und Dimethiconole.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Auch Mischungen von Silikonpolymeren sind geeignet wie zum Beispiel eine Mischung aus Dimethicon und Dimethiconol.

Das erfindungsgemäße Mittel kann in verschiedenen Applikationsformen Anwendung finden, wie beispielsweise in Aerosolzubereitungen als Schaum oder als Spray, desweiteren als Non-Aerosol, welches mittels einer Pumpe oder als "Pump and Spray" zum Einsatz kommt. Der Einsatz in üblichen O/W und W/O Emulsionen ist ebenso möglich wie in Anwendungsformen als Gel, Wachs oder Mikroemulsion.

Das erfindungsgemäße Mittel kann auch als färbendes oder pflegendes Haarbehandlungsmittel wie zum Beispiel als Farbfestiger und Haarspülung formuliert sein.

Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Unter Haarbehandlung soll die Behandlung des menschlichen Kopfhaares vor allem zum Zweck der Herstellung einer Frisur oder zur Pflege der Haare verstanden werden.

Desweiteren ist mit der erfindungsgemäßen Polymerkombination die Herstellung von Konzentraten möglich, welche einen verringerten Wassergehalt und/oder Lösungsmittelgehalt aufweisen. Die Konzentrate werden nach Transport und ggf. Lagerung durch Zugabe der erforderlichen Menge Wasser und/oder Lösungsmittel in ein anwendungsfähiges Haarbehandlungsmittel überführt.

Die nachfolgenden Beispiele sollen den Gegenstand der Erfindung näher erläutern. In den beispielen kann Krambeöl jeweils durch Leindotteröl ersetzt werden und umgekehrt.

Beispiel 1: Schaumfestiger mit starker Festigung

| | |
|---|---|
| 0,25 g | Krambeöl |
| 0,20 g | 2-Pyrrolidon-5-carbonsäure |
| 2,00 g | Polyvinylpyrrolidon |

| | |
|---|---|
| 0,50 g | Chitosan, M = 30.000 bis 70.000 g/mol |
| 0,15 g | Ameisensäure, 85%-ig |
| 0,10 g | Cetyltrimethylammoniunchlorid |
| 0,10 g | Isopropanol |
| 96.70 g | Wasser |
| 100,00 g | |

Beispiel 2: Flüssigfestiger mit starker Festigung

| | |
|---|---|
| 1,00 g | Leindotteröl |
| 1,00 g | Vinylacetat-Crotonsäure-Copolymer, 60%-ige Lösung in Isopropanol/Wasser (ARISTOFLEX® der Firma Hoechst/Deutschland) |
| 2,58 g | Glycerin |
| 50,00 g | Ethanol |
| 0,50 g | Parfümöl |
| 44.92 g | Wasser |
| 100,00 g | |

Beispiel 3: Gelförmiger Festiger mit starker Festigung

| | |
|---|---|
| 0,15 g | Krambeöl |
| 0,10 g | 2-Pyrrolidon-5-carbonsäure |
| 3,00 g | Vinylpyrrolidon-Vinylacetat-Copolymer |
| 1,00 g | Hydroxyethylcellulose |
| 5,00 g | Glycerin |
| 90.75 g | Wasser |
| 100,00 g | |

Beispiel 4: Schaumfestiger für strapaziertes Haar

| | |
|---|---|
| 0,25 g | Leindotteröl |
| 2,00 g | Polyvinylpyrrolidon |
| 0,30 g | α-Hydro-ω-hydroxy-polyoxydimethylsilylen, 13%ig in Cyclodimethylpolysiloxan (Dow Corning Q2 1401 der Firma Dow Corning Europe/Belgien) |
| 5,00 g | 1,2-Propylenglykol |
| 0,10 g | Cetyltrimethylammoniumchlorid |
| 0,10 g | Isopropanol |
| 92.25 g | Wasser |
| 100,00 g | |

Beispiel 5: Schaumfestiger für extra starken Halt

| | |
|---|---|
| 0,15 g | Krambeöl |
| 0,10 g | 2-Pyrrolidon-5-carbonsäure |
| 2,00 g | Polyvinylpyrrolidon |
| 2,00 g | Glukosesirup, 64% Oligosaccharide (C-PUR® 01924 der Firma Cerestar/Belgien) |
| 0,30 g | Polyoxyethylen (4) laurylether |
| 0,10 g | Cetyltrimethylammoniumchlorid |
| 0,10 g | Isopropanol |
| 40,00 g | Ethanol |
| 55.25 g | Wasser |
| 100,00 g | |

**Beispiel 6: Gelförmiger Festiger mit natürlichen Polymeren**

| | |
|---|---|
| 0,15 g | Leindotteröl |
| 5,00 g | Glukosesirup, 64% Oligosaccharide (C-PUR® 01924 der Firma Cerestar/Belgien) |
| 5,00 g | Sorbitsirup |
| 2,00 g | Mutterkornharz (Sclerotium Gum) |
| 87,85 g | Wasser |
| 100,00 g | |

**Beispiel 7: Sprühfestiger zum Fönen**

| | |
|---|---|
| 0,15 g | Krambeöl |
| 0,75 g | Vinylpyrrolidon-Vinylacetat-Copolymer |
| 30,00 g | Ethanol |
| 3,00 g | Propylenglykol |
| 0,75 g | Isopropanol |
| 65,35 g | Wasser |
| 100,00 g | |

**Beispiel 8: Haarwachs**

| | |
|---|---|
| 0,30 g | Leindotteröl |
| 30,00 g | Polyethylenglykol, M = 3.000 g/mol |
| 44,70 g | Polyethylenglykol, M = 600 g/moi |
| 15,00 g | Glycerin |
| 10,00 g | hydriertes Rizinusöl, ethoxyliert mit 45 mol Ethylenoxid |
| 100,00 g | |

**Beispiel 9: Non-Aerosol Haarspray**

| | |
|---|---|
| 0,25 g | Krambeöl |
| 3,00 g | Vinylpyrrolidon-Vinylacetat-Copolymer |
| 96,75 g | Ethanol |
| 100,00 g | |

**Beispiel 10: Pflegendes Shampoo**

| | |
|---|---|
| 0,12 g | Leindotteröl |
| 1,00 g | Vinylpyrrolidon-Vinylacetat-Copolymer |
| 30,00 g | Natriumlaurylethersulfat |
| 7,00 g | Natriumchlorid |
| 3,00 g | Kokosfettsäureamidopropylbetain |
| 0,40 g | hydriertes Rizinusöl, ethoxyliert mit 45 mol Ethylenoxid |
| 0,20 g | Parfüm |
| 58,28 g | Wasser |
| 100,00 g | |

**Beispiel 11: Farbfestiger**

| | |
|---|---|
| 0,2500 g | Krambeöl |
| 3,0000 g | Vinylpyrrolidon-Vinylacetat-Copolymer |
| 50,0000 g | Ethanol |
| 0,2000 g | Parfüm |
| 0,0700 g | 1-Amino-4-(2',3'-dihydroxypropyl)-amino-5-chlor-2-nitrobenzol |
| 0,0500 g | Basic Brown 17 (C.1. 12 251) |
| 0,0023 g | Basic Violett 14 (C.1. 42 510) |
| 0,0100 g | Basic Blue 7 (C.1. 42 595) |
| 46,4177 g | Wasser |
| 100,0000 g | |

**Beispiel 12: Schaumfestiger-Konzentrat**

| | |
|---|---|
| 5,0 g | Leindotteröl |
| 20,0 g | Polyvinylpyrrolidon |
| 10,0 g | Dimethylsiloxan-glykol Copolymer (Belsil DMC 6031 der Firma Wacker/Deutschland |
| 65,0 g | Wasser |
| 100,00 g | |

**Patentansprüche**

1. Verwendung mindestens eines natürlichen Öls, ausgewählt aus Krambeöl und Leindotteröl zur Haarbehandlung.

2. Mittel zur Behandlung von Haaren mit einem Gehalt an mindestens einem natürlichen Öl, welches ausgewählt ist aus Krambeöl und Leindotteröl, in einer geeigneten kosmetischen Grundlage.

3. Mittel nach Anspruch 2, dadurch gekennzeichnet, daß es 0,01 bis 10 Gewichtsprozent des natürlichen Öls enthält.

4. Mittel nach Anspruch 2 oder 3, dadurch gekennzeichnet, daß es zusätzlich 0,01 bis 50 Gewichtsprozent mindestens eines filmbildenden, haarfestigenden Polymeren enthält.